# EUROPÄISCHE PATENTSCHRIFT

(11) **EP 0 979 220 B1**
(45) Veröffentlichungstag und Bekanntmachung des Hinweises auf die Patenterteilung: **19.06.2002**
(21) Anmeldenummer: 98912492.0
(22) Anmeldetag: 20.03.1998
(51) Int. Cl.: C07C 43/13, C07C 41/00

(54) **EINSTUFIGES VERFAHREN ZUR HERSTELLUNG VON ALPHA-HYDROXYETHERN DURCH OXIDATION C=C-UNGESÄTTIGTER VERBINDUNGEN MIT HYDROPEROXIDEN**
SINGLE-STAGE METHOD FOR PRODUCING ALPHA-HYDROXY ETHERS BY OXIDIZING C=C-UNSATURATED COMPOUNDS WITH HYDROPEROXIDES
PROCEDE EN UNE ETAPE DE PRODUCTION D'ALPHA-HYDROXYETHER PAR OXYDATION DE COMPOSES NON SATURES EN C=C AVEC DES HYDROPEROXIDES

(30) Priorität: 24.04.1997 DE 19717265; 20.08.1997 DE 19736121
(43) Veröffentlichungstag der Anmeldung: 16.02.2000
(73) Patentinhaber: SASOL Germany GmbH, 22297 Hamburg (DE)
(72) Erfinder: RÜSCH GEN. KLAAS, Mark, D-48143 Münster (DE); WARWEL, Siegfried, D-52074 Aachen (DE); ZILLMANN, Hans-Martin, D-48159 Münster (DE); KWETKAT, Klaus, D-44534 Lünen (DE)
(74) Vertreter: Schupfner, Gerhard D., Dr. Dipl.-Chem.
(86) Internationale Anmeldenummer: EP9801632
(87) Internationale Veröffentlichungsnummer: WO9847844

(56) Entgegenhaltungen:
- WO-A-96/16033
- GB-A- 1 122 800
- GB-A- 2 252 556
- US-A- 2 808 442

## Beschreibung

Die Erfindung betrifft ein einstufiges Verfahren zur Herstellung von α-Hydroxyethern durch Oxidation C=C-ungesättigter Verbindungen mit Hydroperoxiden in Gegenwart eines ein- oder mehrwertigen Alkohols als Nucleophil und Lösungsmittel, wobei als Katalysatoren Systeme auf Basis von Molybdänverbindungen mit Bortrifluorid oder Aluminiumoxid oder 1,8-Diazabicyclo-[5.4.0]-undec-7-en oder 1,4-Diazabicyclo-[2.2.2]-octan verwendet werden.

α-Hydroxyether, wie in den Formeln (I) und (II) beispielhaft dargestellt, sind Verbindungen von industriellem Interesse. Verbindungen der Formel (I) können in Kosmetika, als Gleitmittel für Kunstharze, in Dispersionsfarben, als Lösungsmittel und als Tensid bzw. Co-Tensid Anwendung finden

Die Verbindungen der Formel (II) gehören zu den Geminitensiden, wie sie in WO 96/16033 beschrieben werden. Diese Verbindungen der Formel (II) dienen entweder als Vorläufer der in WO 96/16033 beschriebenen ionischen und nichtionischen amphiphilen Verbindungen oder können selbst als Emulgatoren, Demulgatoren, Hilfsmittel bei der Metallverarbeitung, Erzgewinnung oder Oberflächenveredelung, als Textilhilfsrnittel oder für das Waschen und Reinigen von Textilien bzw. harten Oberflächen und für das Reinigen und Waschen von Haut und Haar eingesetzt werden.

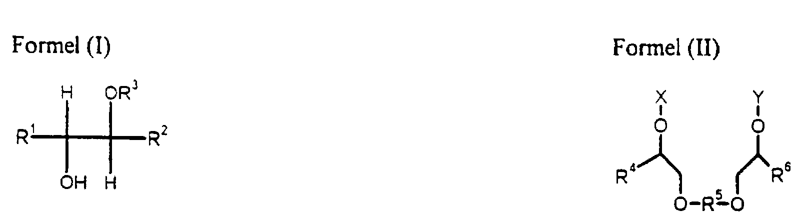

Dabei bedeuten R¹, R², R⁴ und R⁶ unabhängig voneinander gesättigte, unverzweigte oder verzweigte Kohlenwasserstoffreste oder vollständig oder teilweise fluorierte Kohlenwasserstoffreste mit 1 bis 22 Kohlenstoffatomen, vorzugsweise 8 bis 18 Kohlenstoffatomen. Im einzelnen seien die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl, n-Docosyl und ihre verzweigtkettigen Isomeren oder vollständig oder teilweise fluorierten Kohlenwasserstoffreste genannt. R³ steht für ein- oder mehrwertige, lineare oder verzweigte Reste von Alkoholen mit 1 bis 22 Kohlenstoffatomen, die auch ganz oder teilweise fluoriert sein können. Als Beispiele seien Methanol, Ethanol, n- und iso-Propanol, n- und iso-Butanol, Pentanol, Hexanol, Heptanol, n-Octanol, 2-Ethylhexanol, Nonanol, Decanol, Undecanol, Dodecanol, Tridecanol, Tetradecanol, Pentadecanol, Hexadecanol, Heptadecanol, Octadecanol, Nonadecanol, Eicosanol, Heneicosanol, Docosanol, Ethylenglykol, Diethylenglycol, Triethylenglycol, 1,4-Butandiol, Trimethylolpropan, Neopentylglycol, Glycerin und Trifluorethanol und deren Gemische genannt. Bei den längerkettigen Alkoholen sind insbesondere die bezüglich der C-Zahl geradzahligen Verbindungen bevorzugt.
R⁵ bedeutet einen Spacer, bestehend aus einer unverzweigten oder verzweigten Kette mit 2 bis 100 Kohlenstoffatomen, die 0 bis 20 Sauerstoff-, 0 bis 20 Stickstoff-, 0 bis 4 Schwefel- und 0 bis 3 Phosphoratome enthält und die 0 bis 20 funktionelle Seitengruppen, wie z. B. Hydroxyl-, Carbonyl-, Carboxyl-, Amino- und/oder Acylarninogruppen, aufweist. Der Spacer ist in WO 96/16033, die wir hiermit als Referenz einführen, ausführlich beschrieben.

X und Y stehen unabhängig voneinander für Substituenten der Formel XVI

-(C₂H₄O)_{α}(C₃H₆O)_{β}H (XVI)

mit
α = 0 bis 50, vorzugsweise α = 10 bis 30,
β = 0 bis 60, vorzugsweise β = 20 bis 40,
und α + β = 1 bis 100, vorzugsweise α + β = 10 bis 50,
und wobei die Alkoxideinheiten statistisch oder blockweise eingebunden sind und die Reihenfolge beliebig ist
oder für Substituenten der Formel XVII

-(C₂H₄O)_{γ}(C₃H₆O)_{δ}-FR (XVII)

mit jeweils
γ = 0 bis 20, vorzugsweise γ = 0 bis 8,
δ = 0 bis 20, vorzugsweise δ = 0 bis 12,
und γ + δ = 0 bis 40, vorzugsweise γ + δ = 5 bis 20,
wobei FR für einen funktionellen Rest -CH₂-COOM, -SO₃M, -PO(OM)₂,

-C₃H₆-SO₃M

oder

-C(O)-C₂H₃(SO₃M)-CO₂M'

mit M, M' = Alkali, Ammonium-, Alkanofammonium oder
½ Erdalkali, steht.

Der Alkoxylierungsgrad ist jeweils ein Mittelwert und kann jeden beliebigen, auch nicht ganzzahligen Wert in den angegebenen Grenzen einnehmen.

Die Herstellung solcher Hydroxyether aus den zugrundeliegenden Olefinen ist nach dem Stand der Technik ein zweistufiges Verfahren. Zunächst wird das Olefin mit einem geeigneten Oxidationsmittel epoxidiert und anschließend gereinigt. Zur Epoxidation längerkettiger Olefine kommen Persäuren als Oxidationsmittel zum Einsatz. Ethylen und Butadien können an Silberkontakten direkt mit Sauerstoff zu den entsprechenden Epoxiden umgesetzt werden. Die wichtigste Methode zur Herstellung von Propylenoxid ist die Mo-, V-oder Ti-katalysierte Epoxidation von Propylen mit Hydroperoxiden. Obwohl in der Literatur seit langem bekannt ist, daß auch längerkettige Epoxide nach dem sogenannten Halconoder Oxiran-Prozeß epoxidiert werden können [u. a. R.A. Sheldon, J. Mol. Catal., 7 (1980), 107] hat der Prozeß dabei noch keine Verwendung gefunden. In der zweiten Stufe der Herstellung der α-Hydroxyether werden die Epoxide dann mit einem Alkohol üblicherweise in Gegenwart eines Katalysators geöffnet. Für einen technischen Prozeß ist der Einsatz von Epoxiden aufgrund ihres recht hohen Preises oft prohibitiv. Deshalb bestand. hier dringend Bedarf für ein Verfahren, in dem wohlfeile Rohstoffe eingesetzt werden können und die Epoxidzwischenstufe quasi intermediär durchlaufen wird.

Die Oxiranring-Öffnung, die bei kurzkettigen Epoxiden, wie Ethylen- oder Propylenoxid recht leicht verläuft, erfordert mit steigender Kettenlänge immer drastischere Reaktionsbedingungen. Als Katalysatoren kommen sowohl Säuren als auch Basen in Frage, praktisch eingesetzt wurden klassische Brönstedtsäuren, wie H₂SO₄ (R.A. Wohl, Chimie, **28** (1974) 1; DE 38 29 735), eine Vielzahl verschiedener Lewissäuren (u.a. P. Gassmann, T. Guggenheim. J. Am. Chem. Soc., **104** (1982) **5849;** M. Bischoff, U. Zeidler, H. Baumann, Fette, Seifen, Anstrichmittel, **79** (1979) 131), Heteropolysäuren (z. B. Y. Izumi, K. Hayashi, Chem. Lett (1980) 787) und schließlich Al₂O₃ (G.H. Posner, Angew. Chem., **90** (1978) 527) und mit Schwefelsäure behandelte Schichtsilikate (S. Hellbardt, K. Schlandt, W.H. Zech, DE 42 03 077), wobei im letzteren Fall allerdings deutlich erhöhte Temperaturen (>130 °C) notwendig sind.

Bislang sind aus der Literatur nur wenige Versuche bekannt, α-Hydroxyether ausgehend von Olefinen in einer einstufigen Synthese herzustellen. In US 2 808 442 wird die Wolframkatalysierte Direktsynthese von α-Hydroxyethern mit 35- bis 100 %igem Wasserstoffperoxid beschrieben. Das zwangsläufig anwesende Wasser führt dann zur Entstehung vicinaler Diole, so daß man bei der Verwendung von 35 %igem Wasserstoffperoxid von α-Hydroxyethern nurmehr als Nebenprodukten reden kann. Auch Titansilicalithe wurden als Katalysatoren für die Direktsynthese von α-Hydroxyethern aus Olefinen und Alkoholen mit Wasserstoffperoxid eingesetzt (GB 2 252 556), doch bleibt auch hier das Problem der konkurrierenden Ringoffnung durch das anwesende Wasser erhalten.

Aufgabe der vorliegenden Erfindung war es somit, ein Verfahren zur direkten, einstufigen Herstellung von α-Hydroxyethern zu entwickeln, das unter wasserfreien Bedingungen arbeitet und damit die konkurrierende Entstehung von Diol, die als wesentlicher Nachteil der oben beschriebenen Verfahren angesehen werden muß, verhindert.

Die Lösung der erfindungsgemäßen Aufgabe erfolgt durch die Verwendung von
- organischen Hydroperoxiden, ROOH, als Oxidationsmittel
- homogenen oder heterogenen Molybdänverbindungen als erste Katalysatorkomponente
- Bortrifluorid, als stabilisierter Komplex, oder eines Aluminiumoxids oder 1,8-Diazabicyclo-[5.4.0]-undec-7-en oder 1,4-Diazabicyclo-[2.2.2]-octan als zweite Katalysatorkomponente
- ein- oder mehrwertigen Alkoholen als Nucleophil sowie gleichzeitig als Lösungsmittel, was aber die Verwendung anderer Lösungsmittel nicht ausschließt.

Gegenstand der Erfindung ist daher ein einstufiges Verfahren zur Herstellung von α-Hydroxyethern, entsprechend den Formeln (I) und (II), durch die Oxidation olefinischer Substrate mit organischen Hydroperoxiden und die Öffnung des entstandenen Oxiranringes durch ein- oder mehrwertige Alkohole, dadurch gekennzeichnet, daß als Katalysatorsystem Molybdänverbindungen in Kombination mit Bortrifluorid oder Aluminiumoxid oder 1,8-Diazabicyclo-[5.4.0]-undec-7-en oder 1,4-Diazabicyclo-[2.2.2]-octan verwendet werden.

Als Aluminiumoxide, Al₂O₃ seien insbesondere genannt α-Al₂O₃ (Korund), γ-Al₂O₃, oder Hydrate wie α-Al₂O₃ • H₂O (Diaspor), γ-Al₂O₃ (Böhmit), Al₂O₃ • 3 H₂O (Hydrargillit) oder Al₂O₃ • 3 H₂O (Bayerit).

Obwohl die einzelnen Komponenten der Reaktion bekannt sind, ist es überraschend, daß sie sich in einem "Eintopfverfahren" kombinieren lassen und bei der Reaktion selektiv der α-Hydroxyether gebildet wird Die Katalysatorkomponenten beeinflussen sich gegenseitig nicht negativ, es ist sogar zu beobachten, daß BF₃ die Aktivität des Molybdänkatalysators für die Epoxidation erhöht.

Fur das erfindungsgemäße Verfahren geeignete Katalysatorkomponenten für die Epoxidierung sind Molybdänverbindungen, die entweder im Reaktionsgemisch löslich sind, wie Molybdänylacetylacetonat, MoO₂(acac)₂, oder Molybdänhexacarbonyl, Mo(CO)₆, oder Molybdänoxid auf einem Katalysatorträger als heterogener Katalysator. Geeignete Katalysatorträger sind amorphe Alumosilikate oder Zeolithe mit hoher Lewis-Acidität. Der Molybdänkatalysator wird in einer Menge von 0,01 bis 5 mol-%, bevorzugt 0,25 bis 2 mol% und besonders bevorzugt 0,5 bis 1,0 mol-% bezogen auf die zu oxidierende C=C-Doppelbindung eingesetzt.

Als zweite erfindungsgemäße Katalysatorkomponente können Bortrifluorid oder Addukte, wie das Etherat oder das Methanolat eingesetzt werden. Ebenso eignen sich Aluminiumoxid, Al₂O₃, die basische Verbindung 1,8-Diazabicyclo-[5.4.0]-undec-7-en und 1,4-Diazabicyclo-[2.2.2]-octan. Diese Katalysatorkomponenten werden in einer Menge von 0,01 bis 5 mol-%, bevorzugt 0,25 bis 2,0 mol-% und besonders bevorzugt in einer Menge von 0,5 bis 1,0 mol-% bezogen auf die zu oxidierende C=C-Doppelbindung eingesetzt.

Als olefinische Substrate können endständig und / oder innenständig ein- oder mehrfach ungesättigte aliphatische, cyclische oder acyclische Kohlenwasserstoffe wie z. B. Di- und Trimere des Butens oder Tri- und Tetramere des Propens oder ungesättigte Fettsäuren sowie deren Ester eingesetzt werden. Zweckmäßigerweise sollte die Alkoholkomponente bei den Fettsäureestern gleich dem verwendeten Alkohol sein, damit die ebenfalls katalysierte Umesterung nicht zu ungewollten Produktgemischen führt.

Für das erfindungsgemäße Verfahren können ein- oder mehrwertige Alkohole mit primären, sekundären oder tertiären Hydroxylgruppen und beliebiger Kettenlänge eingesetzt werden. Sollen Verbindungen entsprechend der Formel (II) hergestellt werden, so sollten nur primäre Hydroxylgruppen für die Veretherung eingesetzt werden, um unerwünschte Produktgemische zu vermeiden. Der Alkohol und das olefinische Substrat können weitere funktionelle Gruppen, wie zum Beispiel Estergruppen, Carbonylkohlenstoffe, Amide, Ether, enthalten, solange diese in der Reaktion nicht wirkungsvoll als Nucleophile konkurrieren können.

Als Oxidationsmittel für das erfindungsgemäße Verfahren können die kommerziell erhältlichen Hydroperoxide, wie z.B. tert.-Butylhydroperoxid oder Cumolhydroperoxid, verwendet werden. Das Oxidationsmittel wird im Verhältnis 1,0 bis 1,3 bezogen auf die zu oxidierenden Doppelbindungsäquivalente eingesetzt.

Die Reaktion nach dem erfindungsgemäßen Verfahren kann bei Temperaturen beginnend beim Schmelzpunkt des Reaktionsgemisches und endend beim Siedepunkt des Reaktionsgemisches durchgeführt werden. Aus Sicherheitsgründen sollte eine Temperatur von 100 °C nicht überschritten werden. Weiterhin wird die Reaktion in einer Schutzgasatmosphäre und mit von Wasser befreiten Reagenzien durchgeführt. Zur Reaktion werden die Katalysatorkomponenten und Alkohol sowie Olefin gemischt und auf die Reaktionstemperatur erwärmt. Das Hydroperoxid wird dann langsam zudosiert. Nach Beendigung der Reaktion können die Katalysatorkomponenten im einfachsten Fall, beide heterogen, abfiltriert und weiter verwendet werden oder sie müssen, mit Wasser aus dem Produkt entfernt werden. Die entstandenen α-Hydroxyether können i.a. durch Destillation aufgereinigt werden, was aber häufig nicht notwendig ist.

Die nachfolgenden Beispiele sollen die Erfindung näher erläutern, sie jedoch nicht darauf einschränken.

### Beispiele

### 1. Herstellung von 8-Butoxy-7-Tetradecanol

0,03 mol 7-Tetradecen (5,9 g) und 0,3 mmol MoO₂(acac)₂ (98 mg) werden in 30 ml abs. 1-Butanol gelöst, ein Spatel trockenes Molsieb (4Å) zugegeben und auf 90 °C erwärmt. Anschließend gibt man 0,05 ml BF₃-Etherat (≈ 0,3 mmol) und tropfenweise innerhalb von 30 min 0,036 mol abs. tert.-Butylhydroperoxid (3M in Decan, 12 ml) zu. Man läßt noch 16 h nachreagieren, kühlt ab, säuert mit verdünnter Salzsäure an, nimmt in Ether auf und wäscht die organische Phase mit Wasser. Das verbleibende Rohprodukt wird über Na₂SO₄ getrocknet und einer gaschromatographischen Analyse unterworfen. Es enthält neben dem Hautprodukt 8-Butoxy-7-Tetradecanol noch 7,8-Tetradecandiol, 7,8-Epoxytetradecan und nicht umgesetztes 7-Tetradecen. Nach Zusatz eines GC-Standards (Heptansäureethylester) und nach Überführung der freien Carbonsäuren mit CH₂N₂ in die entsprechenden Methylester werden unter Berücksichtigung vorher mittels reiner Substanzen ermittelter Korrekturfaktoren die gebildeten Mengen der Produkte quantitativ bestimmt.
- Ausbeute an 8-Butoxy-7-Tetradecanol:: 60 % der Theorie bez. auf 7-Tetradecen
- Ausbeute an 7,8-Tetradecandiol:: 2 % der Theorie bez. auf 7-Tetradecen
- Ausbeute an 7,8-Epoxytetradecan:: 2 % der Theorie bez. auf 7-Tetradecen
- Umsatz an 7-Tetradecen:: 65 %

### 2. Herstellung von 8-(2-Butoxy)-7-Tetradecanol

Beispiel 1 wird unter Verwendung von 30 ml abs. 2-Butanol wiederholt. Die Nachreaktionszeit beträgt 48 h. Nach gaschromatographischer Analyse erhält man: Ausbeute an 8-(2-Butoxy)-7-Tetradecanol: 55 % der Theorie bez. auf 7-Tetradecen

### 3. Herstellung von 8-(tert-Butoxy)-7-Tetradecanol

Beispiel 1 wird unter Verwendung von 30 ml abs. tert.-Butanol wiederholt. Die Nachreaktionszeit beträgt 48 h. Nach gaschromatographischer Analyse erhält man: Ausbeute an 8-(tert.-Butoxy)-7-Tetradecanol: 48 % der Theorie bez. auf 1-Tetradecen

### 4. Herstellung von 8-Hexoxy-7-Tetradecanol

Beispiel 1 wird unter Verwendung von 30 ml abs. 1-Hexanol wiederholt. Die Nachreaktionszeit beträgt 16 h. Nach gaschromatographischer Analyse erhält man: Ausbeute an 8-Hexoxy-7-Tetradecanol: 58 % der Theorie bez. auf 7-Tetradecen

### 5. Herstellung von 2-Butoxy-1-Tetradecanol/l-Butoxy-2-Tetradecanol

Beispiel 1 wird unter Verwendung von 0,03 mol 1-Tetradecen (5,9 g) wiederholt. Die Nachreaktionszeit beträgt 48 h. Nach gaschromatographischer Analyse erhält man: Ausbeute an 2-Butoxy-1-Tetradecanol/1-Butoxy-2-Tetradecanol:
64 % der Theorie bez. auf 7-Tetradecen

### 6. Herstellung von 2-Butoxy-Cyclohexanol

0,05 mol Cyclohexen (4,1 g) und 0,5 mmol MoO₂(acac)₂ (162 mg) werden in 30 ml abs. 1-Butanol gelöst, ein Spatel trockenes Molsieb (4Å) zugegeben und auf 60 °C erwärmt. Anschließend gibt man 0,08 ml BF₃-Etherat (≈ 0,5 mmol) und tropfenweise innerhalb von 30 min 0,06 mol abs. tert.-Butylhydroperoxid (3M in Decan, 20 ml) zu. Man läßt noch 16 h nachreagieren und arbeitet in der in Beispiel 1 beschriebenen Weise auf. Nach gaschromatographischer Analyse erhält man:
- Ausbeute an 2-Butoxy-Cyclohexanol:: 53 % der Theorie bez. auf Cyclohexen

### 7. Herstellung von 7-Butoxy-8-Hydroxy-octadecansaure-(butylester) bzw.

8-Butoxy-7-Hydroxy-octadecansäure-(butylester)
Beispiel 1 wird unter Verwendung von 0,03 mol Ölsäure (9,6 g, 88 %) wiederholt. Nach Veresterung der freien Carbonsäuren mit CH₂N₂ und gaschromatographischer Analyse erhält man:
Ausbeute an 7-Butoxy-8-Hydroxy-octadecansäurebutylester / 8-Butoxy-7-Hydroxyoctadecansäurebutylester: 37 % der Theorie bez. auf Ölsäure
Ausbeute an 7-Butoxy-8-Hydroxy-octadecansäurebutylester / 8-Butoxy-7-Hydroxyoctadecansäurebutylester: 33 % der Theorie bez auf Ölsäure

### 8. Herstellung von 7-Methoxy-8-Hydroxy-octadecansäuremethylester bzw.

8-Methoxy-7-Hydroxy-octadecansäuremethylester
Beispiel 1 wird unter Verwendung von 0,03 mol Ölsäuremethylester (10,5 g; 85 %) und 30 ml abs. Methanol bei 65 °C wiederholt. Nach gaschromatographischer Analyse erhält man:
Ausbeute an 7-Methoxy-8-Hydroxy-octadecansäuremethylester / 8-Methoxy-7-Hydroxy-octadecansäuremethylester: 65 % der Theorie bez. auf Ölsäure

### 9. Herstellung von 8-(4-Hydroxybutoxy)-7-Tetradecanol

Beispiel 1 wird unter Verwendung von 100 ml abs. 1,4-Butandiol wiederholt. Die Nachreaktionszeit beträgt 16 h. Nach gaschromatographischer Analyse erhält man:
Ausbeute an 8-(4-Hydroxybutoxy)-7-Tetradecanol:
58 % der Theorie bez. auf 7-Tetradecen

### 10. Herstellung von 8-Trifluorethoxy-7-Tetradecanol

Beispiel 1 wird unter Verwendung von 20 ml Trifluorethanol wiederholt. Die Nachreaktionszeit beträgt 16 h. Nach gaschromatographischer Analyse erhält man:
Ausbeute an 8-Trifluorethoxy-7-Tetradecanol:
70 % der Theorie bez. auf 7-Tetradecen

### 11. Herstellung von 8-(2-Methoxyethoxy)-7-Tetradecanol

Beispiel 1 wird unter Verwendung von 30 ml abs. Ethylenglykolmonomethylether wiederholt. Die Nachreaktionszeit beträgt 16 h. Nach gaschromatographischer Analyse erhält man:
Ausbeute an 8-(2-Methoxyethoxy)-7-Tetradecanol:
54 % der Theorie bez. auf 7-Tetradecen

### 12. Herstellung von 8-Butoxy-7-Tetradecanol

Beispiel 1 wird unter Verwendung von 0,3 mmol Mo(CO)₆ (79 mg) wiederholt. Die Nachreaktionszeit beträgt 16 h. Nach gaschromatographischer Analyse erhält man: Ausbeute an 8-Butoxy-7-Tetradecanol: 63 % der Theorie bez. auf 7-Tetradecen

### 13. Herstellung von 8-Butoxy-7-Tetradecanol

Beispiel 1 wird unter Verwendung von 0,036 mol Cumolhydroperoxid (6,9 g; 80 %) wiederholt. Die Nachreaktionszeit beträgt 16 h. Nach gaschromatographischer Analyse erhält man:
Ausbeute an 8-Butoxy-7-Tetradecanol: 50 % der Theorie bez. auf 7-Tetradecen

### 14. Herstellung von 8-Butoxy-7-Tetradecanol

Beispiel 1 wird unter Verwendung eines heterogenen Molybdänkatalysators (nominell 0,3 mmol Mo; 2,9 g) wiederholt. Der heterogene Katalysator wird wie folgt hergestellt:
Zunächst wird ein amorphes Alumosilikat mit 20 Gew.-% SiO₂ bei 550 °C über 16 h im Luftstrom calciniert. 38,4 g dieses Trägers werden in 500 ml 1,4-Dioxan/Wasser (9:1) über Nacht in der Siedehitze mit 4 mmol MoO₂(acac)₂ imprägniert. Anschließend wird das Lösungsmittel am Rotationsverdampfer entfernt und der impragnierte Kontakt wiederum bei 550 °C im Luftstrom aktiviert.
Nach Abfiltrieren des Katalysators und anschließender gaschromatographischer Analyse erhält man:
- Ausbeute an 8-Butoxy-7-Tetradecanol:: 66 % der Theorie bez. auf 7-Tetradecen
- Ausbeute an 7,8-Tetradecandiol:: 2 % der Theorie bez. auf 7-Tetradecen
- Ausbeute an 7,8-Epoxytetradecan:: 18 % der Theorie bez. auf 7-Tetradecen
- Umsatz an 7-Tetradecen:: 94 %

## Patentansprüche

1. Einstufiges Verfahren zur Herstellung von α-Hydroxyethern, entsprechend den Formeln (I) und (II). 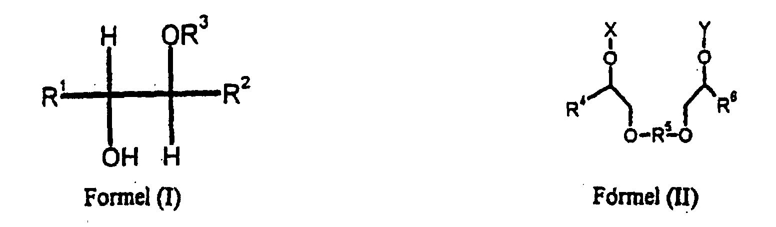 durch die Oxidation olefinischer Substrate mit organischen Hydroperoxiden und die Öffnung des entstandenen Oxiranringes durch ein- oder mehrwertige Alkohole, wobei als Katalysatorsystem Molybdänverbindungen in Kombination mit Bortrifluorid oder Aluminiumoxid oder 1,8-Diazabicyclo-[5.4.0]-undec-7-en oder 1,4-Diazabicyclo-[2.2.2]-octan verwendet werden und wobei R¹, R², R⁴ und R⁶ unabhängig voneinander gesättigte, unverzweigte oder verzweigte Kohlenwasserstoffreste mit 1 bis 22 Kohlenstoffätomen oder vollständig oder teilfluorierte Kohlenwasserstoffreste mit 1 bis 22 Kohlenstoffatomen,
R³ einen ein- oder mehrwertigen, linearen oder verzweigten Rest eines Alkohols mit 1 bis 22 Kohlenstoffatomen, der ganz oder teilweise fluoriert sein kann,
R⁵ einen Spacer, bestehend aus aus einer unverzweigten oder verzweigten Kette mit 2 bis 100 Kohlenstoffatomen, die 0 bis 20 Sauerstoff-, 0 bis 20 Stickstoff-, 0 bis 4 Schwefel- und 0 bis 3 Phosphoratome enthält und die 0 bis 20 Hydroxyl-, Carbonyl-, Carboxyl-, Amino- und/oder Acylaminogruppen, aufweist,
und X und Y unabhängig voneinander Substituenten der Formel XVI
-(C₂H₄O)_{α}(C₃H₆O)_{β}H (XVI)
mit
α = 0 bis 50,
β = 0 bis 60
und α + β = 1 bis 100
und wobei die Alkoxideinheiten statistisch oder blockweise eingebunden sind und die Reihenfolge beliebig ist
oder für Substituenten der Formel XVII
-(C₂H₄O)_{γ}(C₃H₆O)_{δ}-FR (XVII)
mit jeweils
γ = 0 bis 20,
δ = 0 bis 20,
und γ+δ = 0 bis 40
wobei FR für einen funktionellen Rest -CH₂-COOM, -SO₃M, -PO(OM)₂,;
-C₃H₆-SO₃M
oder
-C(O)-C₂H₃(SO₃M)-CO₂M'
mit M, M' = Alkali, Ammonium-, Alkanolammonium oder ½ Erdalkali, steht und wobei die Alkoxideinheiten ebenfalls statistisch oder blockweise eingebunden sind und die Reihenfolge beliebig ist, bedeuten.

2. Verfahren nach Anspruch 1,
**dadurch gekennzeichnet,**
**daß** R¹, R², R⁴ und R⁶ unabhängig voneinander gesättigte, unverzweigte oder verzweigte Kohlenwasserstoffreste oder vollständig oder teilweise fluorierte Kohlenwasserstoffreste mit 8 bis 18 Kohlenstoffatomen bedeuten.

3. Verfahren nach Anspruch 1,
**dadurch gekennzeichnet,**
**daß** man den ein- oder mehrwertigen Alkohol als Lösungsmittel einsetzt.

4. Verfahren nach einem der vorhergehenden Ansprüche,
dadurch gekennzeichent,
daß man unter Wasserausschluß arbeitet.

5. Verfahren nach einem der vorhergehenden Ansprüche
**dadurch gekennzeichnet,**
**daß** man lösliche Molybdänverbindungen verwendet.

6. Verfahren nach einem der vorhergehenden Ansprüche
**dadurch gekennzeichnet,**
**daß** man MoO₂(acac)₂ oder Mo(CO)₆ verwendet.

7. Verfahren nach einem der Ansprüche 1 bis 3,
**dadurch gekennzeichnet,**
**daß** man Molybdänoxid auf einem Katalysatorträger einsetzt.

8. Verfahren nach Anspruch 7,
**dadurch gekennzeichnet,**
**daß** man als Katalysatorträger amorphe Alumosilikate oder Zeolithe einsetzt.

9. Verfahren nach einem der vorhergehenden Ansprüche,
**dadurch gekennzeichnet,**
**daß** man ungesättigte Fettsäuren oder Fettsäureester oder Di- und Trimere des Butens oder Tri- und Tetramere des Propens einsetzt.

## Claims

1. A single-stage process for the production of α-hydroxy ethers according to formulae (I) and (II) 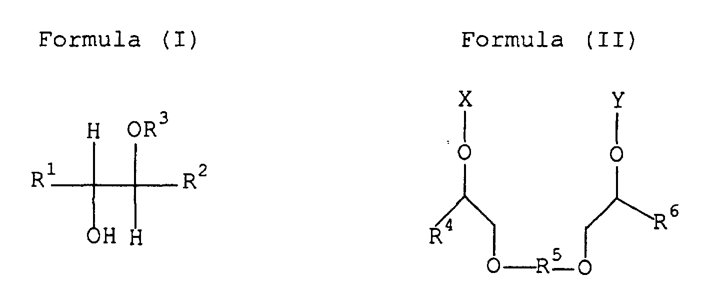 by oxidation of olefinic substrates with organic hydroperoxides and opening of the resultant oxirane ring by mono- or polyhydric alcohols, wherein molybdenum compounds in combination with boron trifluoride or alumina or 1,8-diazabicyclo-[5.4.0]-undec-7-ene or 1,4-diazabicyclo-[2.2.2]-octane are used as a catalyst system, and wherein
**R**^{**1**}**, R**^{**2**}**, R**^{**4**}**,** and **R**^{**6**}**,** independently of one another, are saturated, unbranched or branched hydrocarbon radicals having 1 to 22 carbon atoms, or are completely or partially fluorinated hydrocarbon radicals having 1 to 22 carbon atoms,
**R**^{**3**} represents a mono- or polyhydric, linear or branched radical of an alcohol having 1 to 22 carbon atoms which may be fluorinated wholly or in part,
**R**^{**5**} is a spacer consisting of an unbranched or branched chain with 2 to 100 carbon atoms which contains 0 to 20 oxygen atoms, 0 to 20 nitrogen atoms, 0 to 4 sulfur atoms, and 0 to 3 phosphorus atoms and which has 0 to 20 hydroxyl, carbonyl, carboxyl, amino and/or acylamino groups, and
**X** and **Y**, independently of one another, are substituents according to formula XVI
- (C₂H₄O)_{α}(C₃H₆O)_{β}H **(XVI)**
where
α = 0 to 50
β = 0 to 60
and α + β = 1 to 100
and wherein the alkoxide units are incorporated randomly or blockwise and the sequence is optional,
or are substituents according to formula XVII
- (C₂H₄O)_{γ}(C₃H₆O)_{δ}-FR **(XVII)**
where each
γ = 0 to 20
δ = 0 to 20
and γ + δ = 0 to 40
and **FR** represents a functional radical
-CH₂-COOM, -SO₃M, -P O (OM)₂,
-C₃H₆-SO3M, or
-C(O) -C₂H₃(SO₃M)-CO₂M', wherein M, M' are equal to alkali, ammonium, alkanol ammonium, or ½ alkaline earth metal, and the alkoxide units, too, are incorporated randomly or blockwise and the sequence is optional.

2. A process as claimed in claim 1, **characterized in that**
**R**^{**1**}**, R**^{**2**}**, R**^{**4**}**,** and **R**^{**6**}**,** independently of one another, are saturated, unbranched or branched hydrocarbon radicals, or are completely or partially fluorinated hydrocarbon radicals having 8 to 18 carbon atoms.

3. A process as claimed in claim 1, **characterized in that**
the mono- or polyhydric alcohol is used as a solvent.

4. A process as claimed in any one of the preceding claims, **characterized in that**
the process is performed in the absence of water.

5. A process as claimed in any one of the preceding claims, **characterized in that**
soluble molybdenum compounds are employed.

6. A process as claimed in any one of the preceding claims, **characterized in that**
MoO₂(acac)₂ or Mo(CO)₆ is employed.

7. A process as claimed in any one of claims 1 to 3,
**characterized in that**
molybdenum oxide on a catalyst support is employed.

8. A process as claimed in claim 7,
**characterized in that**
amorphous alumino-silicates or zeolites are used as catalyst supports.

9. A process as claimed in any one of the preceding claims, **characterized in that**
unsaturated fatty acids or fatty acid esters or di- and trimers of butene or tri- and tetramers of propene are employed.

## Revendications

1. Procédé en une étape pour la préparation d'α-hydroxyéthers, correspondant aux formules (I) et (II) 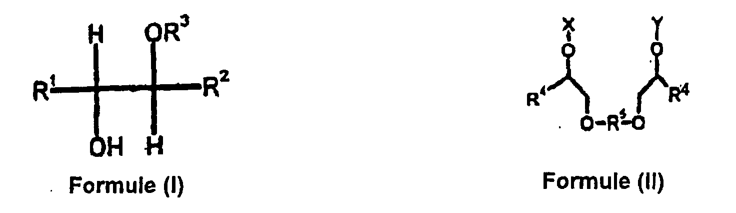 par l'oxydation de substrats oléfiniques avec des hydroperoxydes organiques et l'ouverture du cycle oxiranne formé par des alcools monovalents ou polyvalents, dans lequel des composés du molybdène sont utilisés en combinaison avec du trifluorure de bore ou de l'oxyde d'aluminium ou du 1,8-diazabicyclo-[5.4.0]-undéc-7-ène ou du 1,4-diazabicyclo-[2.2.2]-octane comme système catalyseur et R¹, R², R⁴ et R⁶ signifiant, indépendamment les uns des autres, des radicaux hydrocarbonés saturés, non ramifiés ou ramifiés comprenant 1 à 22 atomes de carbone ou des radicaux hydrocarbonés complètement. ou partiellement fluorés comprenant. 1 à 22 atomes de carbone,
R³ signifiant un radical monovalent ou polyvalent, linéaire ou ramifié d'un alcool comprenant 1 à 22 atomes de carbone, qui peut avoir été complètement ou partiellement fluoré,
R⁵ signifiant un espaceur, constitué d'une chaîne non ramifiée ou ramifiée comprenant 2 à 100 atomes de carbone, qui contient 0 à 20 atomes d'oxygène, 0 à 20 atomes d'azote, 0 à 4 atomes de soufre et 0 à 3 atomes de phosphore et qui présente 0 à 20 groupements hydroxyle, carbonyle, carboxyle, amino et/ou acylamino,
et X et Y signifiant, indépendamment l'un de l'autre, des substituants de formule XVI
- (C₂H₄O)_{α}(C₃H₆O)_{β}H (XVI)
avec
α = 0 à 50,
β = 0 à 60,
et α + β = 1 à 100
et dans laquelle les unités alcoxyde sont liées statistiquement ou par blocs et l'ordre est quelconque
ou signifiant des substituants de formule XVII
- (C₂H₄O)_{γ}(C₃H₆O)_{δ}FR (XVII)
avec à chaque fois
y = 0 à 20,
δ = 0 à 20,
et γ + δ = 0 à 40
dans laquelle FR signifie un radical fonctionnel -CH₂-COOM, -SO₃M, -PO(OM)₂, -C₃H₆-SO₃M ou -C (O)-C₂H₃(SO₃M)-CO₂M'
avec M, M' = un métal alcalin, ammonium, alcanolammonium ou demi métal alcalino-terreux et dans laquelle les unités alcoxyde sont également liées statistiquement ou par blocs et l'ordre est quelconque.

2. Procédé selon la revendication 1, **caractérisé en ce que** R¹, R², R⁴ et R⁶ signifient, indépendamment les uns des autres, des radicaux hydrocarbonés saturés, non ramifiés ou ramifiés ou des radicaux hydrocarbonés complètement ou partiellement fluorés comprenant 8 à 18 atomes de carbone.

3. Procédé selon la revendication 1, **caractérisé en ce qu'**on utilise l'alcool monovalent ou polyvalent comme solvant.

4. Procédé selon l'une quelconque des revendications précédentes, **caractérisé en ce qu'**on travaille en excluant l'eau.

5. Procédé selon l'une quelconque des revendications précédentes, **caractérisé en ce qu'**on utilise des composés solubles du molybdène.

6. Procédé selon l'une quelconque des revendications précédentes, **caractérisé en ce qu'**on utilise du MoO₂(acac)₂ ou du Mo(CO)₆.

7. Procédé selon l'une quelconque des revendications 1 à 3, **caractérisé en ce qu'**on utilise de l'oxyde de molybdène sur un support de catalyseur.

8. Procédé selon la revendication 7, **caractérisé en ce qu'**on utilise comme support de catalyseur des aluminosilicates amorphes ou des zéolithes.

9. Procédé selon l'une quelconque des revendications précédentes, **caractérisé en ce qu'**on utilise des acides gras insaturés ou des esters d'acide gras insaturés ou des dimères et des trimères du butène ou des trimères et des tétramères du propène.
